# FASCICULE DE BREVET EUROPEEN

(11) **EP 1 092 728 B1**
(45) Date de publication et mention de la délivrance du brevet: **24.05.2006**
(21) Numéro de dépôt: 00480090.0
(22) Date de dépôt: 05.10.2000
(51) Int. Cl.: C07K 14/78, A61K 47/48, A61K 47/42, A61K 33/00, A23L 1/03

(54) **Complexe à base d'acide orthosilicique biologiquement assimilable, se présentant sous forme solide, stable et concentrée, et procédé de préparation**
Fester, stabiler und konzentrierter biologisch verdaulicher Komplex von Orthokieselsäure und Prozess zur dessen Herstellung
Biologically digestable complex of orthosilicic acid in a solid, stable and concentrated form and process for its preparation

(30) Priorité: 15.10.1999 FR 9913062
(43) Date de publication de la demande: 18.04.2001
(73) Titulaire: EXSYMOL S.A.M., MC-98000 Monte Carlo (MC); Seguin, Marie-Christine, 98000 Monaco (MC); Gueyne, Jean, 98000 Monaco (MC)
(72) Inventeur: Seguin, Marie-Christine, 98000 Monaco (MC); Gueyne, Jean, 98000 Monaco (MC)
(74) Mandataire: Bonneau, Gérard

(56) Documents cités:
- WO-A-95/21124
- FR-A- 2 122 529
- DATABASE WPI Section Ch, Week 199735 Derwent Publications Ltd., London, GB; Class A82, AN 1997-380816 XP002141167 & JP 09 164761 A (KONICA CORP), 24 juin 1997 (1997-06-24)

## Description

### Domaine technique :

La présente invention concerne un complexe à base d'acide orthosilicique biologiquement assimilable, se présentant sous forme solide, stable et concentrée, et un procédé de préparation dudit complexe.

### Etat de la technique :

Le silicium est un élément essentiel de la vie. Il a à la fois un rôle structural en tant qu'élément constitutif des complexes protéines-glycosaminoglycanes de la matrice des tissus conjonctifs, et un rôle métabolique sur la croissance et l'ostéogenèse (il favorise le processus de minéralisation de l'os).
Le silicium est donc indispensable pour un développement normal des os et des tissus conjonctifs.

On a également démontré que le silicium sous forme d'acide orthosilicique Si(OH)₄ avait une forte affinité pour les ions Al³⁺ et favorisait leur élimination. Il pourrait de ce fait contrer les effets toxiques de l'aluminium sur les os et le cerveau, en particulier dans le cas des maladies neurologiques dégénératives telles que la maladie d'Alzheimer.

Ces données suggèrent qu'un apport ponctuel en silicium peut se révéler très utile, en période de croissance par exemple, ou dans certaines situations pathologiques.

Mais il est également important d'éviter des situations de carence en cet élément : divers travaux effectués chez l'homme ont montré que la teneur en silicium diminue avec l'âge, dans la peau et les parois artérielles, principalement à cause de la chute de l'absorption gastro-intestinale du silicium au cours des années.

Le gros problème du métabolisme du silicium vient de la difficulté à l'assimiler. Bien que le silicium soit un élément très abondant, il existe à l'état naturel essentiellement sous forme de minéraux insolubles dans l'eau (oxydes cristallins (silices et silicates) ou amorphes (argiles, opales,...), qui ne constituent pas une source de silicium assimilable par l'organisme.
De même, le silicium tel qu'il se présente dans notre alimentation, c'est à dire majoritairement sous forme d'aluminosilicates ou de silice, est faiblement biodisponible. Les techniques actuelles de purification et de raffinage des produits végétaux (céréales et fibres alimentaires constituent la principale source de silicium), qui ont tendance à éliminer les parties riches en silicium (les enveloppes), ont encore réduit cet apport.

Il semble maintenant acquis qu'une forme biologiquement active de silicium doit être hydrosoluble, et que cette solubilité dépend de la quantité de groupements OH libres (fonctions silanol) présents sur l'atome de silicium.
Dans le sang et les liquides extravasculaires, le silicium est présent de façon physiologique sous forme hydratée d'acide orthosilicique (Si(OH)₄), non dissocié au pH de l'organisme, et en quasi totalité sous forme libre.
Des études ont par ailleurs montré que seul l'acide orthosilicique monomère, ou oligomère (très faiblement polymérisé), est capable de franchir la barrière intestinale (P.Creach, J.Adrian, Méd et Nut. 1990, 26 (2)).

La présence sur l'atome de silicium de groupements OH libres est aussi étroitement liée à son rôle biologique. Ces fonctions silanols peuvent en effet établir des liaisons covalentes ou hydrogène avec les amides, l'eau, les alcools, cétones, esters. Ainsi, dans les milieux biologiques peuvent se former des liaisons avec les phospholipides membranaires, les protéines, les glycosaminoglycanes ou les polysaccharides.

Il est donc nécessaire de concevoir un apport en silicium tel que cet élément soit sous forme d'acide silicique ou une forme aussi proche que possible.

L'acide orthosilicique monomère en solution est relativement stable jusqu'à une concentration d'environ 10⁻⁴ Mol.l⁻¹, mais lorsque la concentration croît, les molécules de Si (OH)₄ s'associent pour constituer des oligomères puis des polymères d'acide orthosilicique (formation de liaisons siloxanes Si-O-Si) jusqu'à former des solutions colloïdales ou des gels de silice, formes peu solubles ou insolubles dans l'eau, pour lesquelles la biodisponibilité du silicium est très faible.
L'apport d'acide orthosilicique sous la forme de solutions très diluées stables s'accorde mal avec une supplémentation journalière en silicium, et rend notamment impossible son incorporation, à des doses suffisamment élevées, dans des nutriments, des compositions diététiques, cosmétiques ou thérapeutiques.

On dispose actuellement, comme sources de silicium biologiquement actif concentré, de composés organosiliciés présentant plusieurs groupements hydroxyles libres, pouvant se présenter sous forme de solutions ou sous forme solide (brevets EP- 0 289 366 et FR 2 761 074).

On dispose aussi de solutions plus concentrées d'acide orthosilicique chimiquement stabilisé par un pH très acide qui s'oppose à la polymérisation par hydrolyse des fonctions siloxanes Si-O-Si (brevet JP 58176115).

Le brevet WO 95/21124 propose également des préparations concentrées d'acide orthosilicique stabilisé par un composé stabilisant. L'acide orthosilicique en solution est complexé à la choline, mais une stabilité tout à fait satisfaisante ne peut être obtenue qu'en maintenant un pH fortement acide.

De telles solutions acides ne permettent pas d'obtenir une assimilation optimale car lors de l'ingestion, les conditions de pH ne sont pas maintenues (pH physiologique). Une part importante de l'acide orthosilicique polymérise avant d'avoir été assimilé.

On a aussi utilisé, dans le cadre d'une étude médicale, de l'acide silicique colloïdale en application topique combinée à une prise orale, pour améliorer l'état de peaux âgées, de cheveux fragiles et d'ongles cassants (Lassus A., Journal of International Medical Research 1993, 21; 209-215). Cependant, la biodisponibilité de cette forme est faible du fait de la présence de nombreuses liaisons siloxanes.

### Exposé de l'invention :

C'est pourquoi un but de l'invention est de proposer une forme d'acide orthosilicique assimilable concentrée et stable.

De plus, pour être assimilable par voie orale, l'acide orthosilicique doit être soluble et chimiquement stable aux différents pH physiologiques c'est à dire en milieu acide, neutre ou légèrement alcalin.
C'est pourquoi un autre but de la présente invention est l'obtention d'une forme d'acide orthosilicique stable à ces différents pH.

Enfin, un autre objectif était d'obtenir une forme solide pouvant être incorporée dans des compositions diététiques, cosmétiques ou thérapeutique, ainsi que dans des compléments nutritionnels, se présentant sous des formes non aqueuses : gélules, granulés ou cachets destinés à être administré par voie orale.

Ces objectifs peuvent être atteints en complexant l'acide orthosilicique à un polypeptide qui joue un rôle de stabilisant en établissant des liaisons hydrogène avec l'acide orthosilicique, ce qui empêche la formation de liaisons siloxanes Si-O-Si et la polymérisation de l'acide orthosilicique.

On obtient ainsi une forme solide (poudre) d'acide orthosilicique stable qui peut, après ingestion, se solubiliser dans les liquides biologiques pour former une espèce soluble d'acide orthosilicique assimilable et biologiquement active (monomère ou oligomère).

Au delà de l'excellente stabilité de la forme solide concentrée, une caractéristique importante de l'invention réside dans sa capacité à rester stable lors de son passage dans le tractus gastro-intestinal, et ce malgré l'existence de différents pH physiologiques favorables à sa polymérisation.
Ceci s'explique par la nature particulièrement forte de l'interaction existant entre le silicium et le polypeptide. Lors de sa dissolution, le complexe entre la chaîne polypeptidique et les fonctions OH portées par l'atome de silicium persiste; la cohésion de ce complexe est liée à la formation d'un ensemble de liaisons faibles (hydrogène) et probablement à une organisation structurale de la chaîne polypeptidique.

On notera que dans la perspective de préparer des formes retard, des peptides à activité pharmacologique ont été associés à des minéraux naturels contenant du silicium, ou à des minéraux synthétiques à base d'acide silicique (brevet BE. N° 778.239). La caractéristique commune à tous les composés silicés utilisés étant de former une suspension colloïdale propre à servir de support aux peptides; il est clair que le problème technique abordé n'a aucun rapport avec celui de la présente invention.

### Description de l'invention :

L'objet de la présente invention est donc de proposer un complexe à base d'acide orthosilicique biologiquement assimilable caractérisé en ce que l'acide orthosilicique est complexé à un polypeptide et en ce qu'il se présente sous forme solide, stable et concentrée.

L'objet de la présente invention est également de proposer un procédé de préparation dudit complexe à base d'acide orthosilicique, comprenant les étapes suivantes :
- le polypeptide est dissous dans 1 volume d'eau distillée
- le pH est ajusté entre 2 et 4
- on ajoute sous agitation, dans une proportion allant de 1/4 de volume à 1 volume, un alcool soluble dans l'eau, préférablement l'éthanol
- un précurseur hydrolysable d'acide orthosilicique, de préférence un tetraalcoxysilane, est introduit au goutte à goutte
- le mélange est maintenu sous agitation jusqu'à hydrolyse complète du précurseur
- l'alcool et l'eau sont évaporés.
On obtient ainsi un complexe acide orthosilicique-polypeptide se présentant sous la forme d'un solide pulvérulent légèrement coloré, avec une teneur en silicium pouvant aller jusqu'à 10%. Préférablement, la teneur en silicium est comprise entre 1 et 5%.

Suivant un mode de réalisation avantageux, le polypeptide est un hydrolysat de protéines d'origine animale ou végétale.

En effet, les polypeptides obtenus après une hydrolyse poussée se sont avérés être les plus appropriés pour la réalisation des buts de l'invention.

Les polypeptides de haut poids moléculaire, et notamment certaines protéines, se sont révélés être de médiocre stabilisants. Ceci s'explique peut être par la structure tertiaire de ces protéines qui n'est pas favorable à un agencement stabilisant avec l'acide orthosilicique.

Selon un mode préféré de réalisation de l'invention, le polypeptide est un hydrolysat de collagène, tel que le collagène de porc ou de poisson.

En effet, parmi les hydrolysats de protéines d'origine animale, l'hydrolysat de collagène est particulièrement approprié dans la mesure où le collagène possède une structure particulièrement favorable à la formation d'un complexe avec l'acide orthosilicique. Le silicium est d'ailleurs fortement associé au collagène in vivo (Carlisle EM, The Science of Total Environment, 1988, vol.73, 95-106).

Enfin, un autre but de la présente invention est de proposer des compositions cosmétiques ou pharmaceutiques ainsi qu'un complément nutritionnel comprenant, en association avec tout excipient approprié, un tel complexe.

Parmi les multiples utilisations de ces compositions et complément nutritionnel on peut citer : les cas de carence en silicium, la nécessité d'un apport accru chez la femme enceinte, l'enfant en période de croissance ou chez les personnes âgées où la capacité d'assimilation du silicium est diminuée en même temps que la fixation tissulaire, la régénération et le renforcement de la peau, des cheveux, des ongles, la prévention et le traitement de maladies telles que l'ostéoporose ou l'athérosclérose.

Les exemples qui suivent ont pour but d'illustrer de manière non limitative la présente invention.

### Exemple 1 : Obtention d'une poudre d'acide orthosilicique à 1,5% en Silicium au moyen d'une gélatine alimentaire hydrolysée d'origine porcine.

90 g d'hydrolysat de protéines porcines sont dissous sous agitation dans 250 ml d'eau distillée. Le pH est ajusté entre 3 et 4 avec une solution d'acide chlorhydrique 6 N.
On rajoute ensuite lentement, sous agitation, 270 ml d'éthanol absolu. On obtient une solution jaune-orangée homogène, très légèrement opalescente.
Du tetraéthoxysilane (11,25 g) est alors ajouté au mélange au goutte à goutte, sous agitation.
La solution est maintenue sous agitation jusqu'à totale hydrolyse du précurseur.
L'alcool et l'eau sont ensuite évaporés sous pression réduite.
On obtient 92 g d'un solide pulvérulent légèrement coloré (jaunâtre), dont la teneur résiduelle en eau est proche de 5,5%.

### Exemple 2 : Obtention d'une poudre d'acide orthosilicique au moyen d'hydrolysat de protéines de blé.

180 g d'hydrolysat de protéines de blé sont dissous sous agitation, dans 500 g d'eau distillée. On ajoute 400 ml d'éthanol absolu. A l'aide d'une solution d'acide chlorhydrique dilué, le milieu est acidifié.

23 g de tétraéthoxysilane sont alors ajoutés au mélange, au goutte à goutte sous agitation. La solution est maintenue sous agitation jusqu'à hydrolyse complète du tétraéthoxysilane.
L'éthanol et l'eau sont ensuite éliminés par distillation sous pression réduite.
On obtient un solide légèrement coloré, qui peut être broyé pour former une poudre parfaitement soluble dans l'eau avec une teneur en silicium d'environ 2,5 %.

### Exemple 3 : Etude de stabilité

Cette étude a été réalisée à 37°C (±3°C) sur des solutions aqueuses à 0,075 % en Si à pH stomacal (1,2) et pH 7,5 (pH intestinal). Le critère de stabilité est la solubilité du silicium.

| | pH 1,2 | pH 7,5 |
|---|---|---|
| Teneur en Si soluble à t0 (g / Kg) | 0,75 | 0,75 |
| Teneur en Si soluble à t+8h (g / Kg) | 0,75 | 0,6 |
| Teneur en Si soluble à t+24 h (g / Kg) | 0,7 | 0,45 |

### Exemple 4 : Test de diffusion sur duodénum

On mesure et on compare l'assimilation, au niveau de la paroi duodénale, de plusieurs formes de silicium en solution aqueuse (0,6 % en Si) : une solution obtenue par dissolution d'une poudre d'acide orthosilicique stabilisé par un polypeptide de collagène, une solution comprenant de l'acide orthosilicique non stabilisé et une solution de silicate de sodium .

Des rats Sprague Dawley sont sacrifiés par injection intracardiaque de pentobarbital disodique. Immédiatement après, le duodénum de chaque rat est prélevé, vidé et lavé avec une solution de NaCl (0,9 %). Après introduction d'environ exactement 1 ml des solutions d'actif, les organes sont ligaturés avec du fil de coton et placés dans 10 ml d'une solution isotonique tamponnée à 37°C. A différents temps de l'étude, 1 ml de la solution isotonique tamponnée est prélevé et filtré.
La concentration en silicium soluble est évaluée par absorption atomique.

Les solutions d'actifs pour duodénum sont ramenées à pH=8,5.

### Résultats:

| Acide orthosilicique complexé | | Silicate de Na | | Acide orthosilicique non stabilisé | |
|---|---|---|---|---|---|
| Temps (min) | % de Silicium ayant diffusé | Temps (min) | % de Silicium ayant diffusé | Temps (min) | % de Silicium ayant diffusé |
| 0 | 0 | 0 | 0 | 0 | 0 |
| 23 | 1,55 | 24 | 0,83 | 24 | 0,67 |
| 31 | 2,07 | 28 | 0,91 | 28 | 0,99 |
| 39 | 2,59 | | | 34 | 1 |
| 53 | 3,5 | 52 | 1,38 | 48 | 1,16 |
| 57 | 3,76 | 56 | 1,47 | 56 | 1,54 |
| | | 60 | 1,55 | 60 | 1,75 |
| 65 | 4,28 | 66 | 1,67 | 68 | 1,86 |
| 73 | 5,8 | 70 | 1,75 | 70 | 2,08 |
| 74 | 4,87 | 74 | 1,83 | 74 | 2,13 |
| 82 | 5,39 | 86 | 2,07 | 85 | 2,54 |
| 90 | 6,2 | 90 | 2,15 | 90 | 2,67 |

Les résultats montrent que la forme acide orthosilicique complexé à un polypeptide a diffusé 2 fois plus que l'acide orthosilicique non stabilisé et 3 fois plus que le silicate de sodium (ces dernières formes ne diffusant que faiblement).
Seule la forme complexée a diffusé et a donc été assimilée.
Ceci confirme que l'acide orthosilicique biologiquement assimilable doit être soluble dans l'eau et stable au pH tissulaire.

### Exemple 5 : complément nutritionnel destiné à favoriser la pousse des phanères, des ongles et des cheveux, se présentant sous la forme d'une poudre pour solution buvable.

| | |
|---|---|
| Protéines végétales | 90 g |
| Vitamine E | 100 mg |
| Fer | 60 mg |
| Zinc | 14 mg |
| Magnésium | 450 mg |
| Silicium | 70 mg |
| sous forme de poudre d'acide silicique complexé à 1,5 % en Si | |
| Excipients (bicarbonate de sodium, saccharose) qsp | 100 g |

Ce complément se présente sous la forme d'une poudre pour solution buvable en sachets de 7 g. La posologie est de 1 sachet par jour soit un apport de 5 mg de silicium sous forme de complexe d'acide silicique pulvérulent à 1,5 % en silicium (ce qui correspond à 0,33 g de complexe).

### Exemple 6 : Composition thérapeutique dont les indications sont les suivantes : ostéoporose, arthrose, artériosclérose des membres inférieurs.

| | |
|---|---|
| Silicium | 750 mg |
| sous forme de poudre d'acide silicique complexé à 1,5% en Si | |
| Lactose qsp | 100 g |

Cette composition peut se présenter sous forme de gélules de 0,33 g chacune soit 2,47 mg de silicium sous forme de complexe d'acide silicique à 1,5 % (soit 0,165 g de complexe). La prise journalière recommandée est de 2 gélules.

## Revendications

1. Complexe à base d'acide orthosilicique, **caractérisé en ce que** l'acide orthosilicique est complexé à un polypeptide et **en ce que** ledit complexe se présente sous forme solide, stable, concentrée et biologiquement assimilable.

2. Complexe selon la revendication 1, **caractérisé en ce que** ledit polypeptide est un hydrolysat de protéines d'origine animale ou végétale.

3. Complexe selon l'une des revendications 1 ou 2, **caractérisé en ce que** ledit polypeptide est un hydrolysat de collagène, tel que le collagène de porc ou de poisson.

4. Procédé de préparation du complexe selon l'une des revendications 1 à 3, **caractérisé en ce qu'**il comprend les étapes suivantes :
- le polypeptide est dissous dans 1 volume d'eau distillée
- le pH est ajusté entre 2 et 4
- on ajoute sous agitation, dans une proportion allant de 1/4 de volume à 1 volume, un alcool soluble dans l'eau, préférablement l'éthanol
- un précurseur hydrolysable d'acide orthosilicique, de préférence un tétraalcoxysilane, est introduit au goutte à goutte
- le mélange est maintenu sous agitation jusqu'à hydrolyse complète du précurseur
- l'alcool et l'eau sont évaporés.

5. Composition cosmétique ou pharmaceutique, **caractérisée en ce qu'**elle comprend, en association avec tout excipient approprié, un complexe à base d'acide orthosilicique biologiquement assimilable selon l'une des revendications 1 à 3.

6. Complément nutritionnel **caractérisé en ce qu'**il comprend, en association avec tout excipient alimentairement approprié, un complexe à base d'acide orthosilicique biologiquement assimilable selon l'une des revendications 1 à 3.

## Claims

1. Complex containing orthosilicic acid, **characterized in that** the orthosilicic acid is complexed with a polypeptide, and **in that** it is under a solid, stable, concentrated and biologically assimilable form.

2. Complex according to claim 1, **characterized in that** the said polypeptide is a protein hydrolysate of animal or vegetable origin.

3. Complex according to any one of claims 1 or 2, **characterized in that** the said polypeptide is a collagen hyrolysate, such as swine or fish collagen.

4. Process of preparation of the complex according to any of claims 1 to 3, **characterized in that** it comprises the following steps:
- the polypeptide is dissolved in 1 volume of distillated water;
- the pH is adjusted between 2 and 4;
- in a proportion comprising of between ¼ and 1 volume, an alcohol soluble in water, preferably ethanol, is added under stirring;
- a hydrolysable precursor of orthosilicic acid, preferably tetraalkoxysilane, is introduced dropwise;
- the mixture is maintained under stirring until whole hydrolysis of the precursor; and
- alcohol and water are evaporated.

5. Cosmetic or pharmaceutical composition **characterized in that** it includes, in association with any suitable excipient, a complex containing biologically assimilable orthosilicic acid according to any one of claims 1 to 3.

6. Nutritional supplement **characterized in that** it includes, in association with any dietary suitable excipient, a complex containing biologically assimilable orthosilicic acid according to any one of claims 1 to 3.

## Patentansprüche

1. Ein Komplex auf der Basis von Orthokieselsäure, **dadurch gekennzeichnet, dass** die Orthokieselsäure mit einem Polypeptid komplexiert ist, und **dadurch**, dass der Komplex in einer festen, stabilen, konzentrierten und biologisch assimilierbaren Form vorliegt.

2. Komplex gemäß Anspruch 1, **dadurch gekennzeichnet, dass** das Polypeptid ein Hydrolysat aus tierischen oder pflanzlichen Proteinen ist.

3. Komplex gemäß einem der Ansprüche 1 oder 2, **dadurch gekennzeichnet, dass** das Polypeptid ein Hydrolysat aus Kollagen, wie etwa Schweinekollagen oder Fischkollagen, ist.

4. Ein Verfahren zur Zubereitung des Komplexes gemäß einem der Ansprüche 1 bis 3, **dadurch gekennzeichnet, dass** es die folgenden Schritte umfasst:
- Auflösen des Polypeptids in 1 Volumen distilliertem Wasser
- Einstellen des pH-Werts zwischen 2 und 4
- Beimischen unter Rühren eines wasserlöslichen Alkohols, vorzugsweise Ethanol, in einem Verhältnis von 1/4 eines Volumens bis zu 1 Volumen
- Tröpfchenweises Einführen eines hydrolysierbaren Orthokieselsäure-Präkursors, vorzugsweise ein Tetraalkoxysilan
- Aufrechterhalten der Mischung unter Rühren bis zur vollständigen Hydrolyse des Präkursors
- Verdampfen des Wassers und des Alkohols.

5. Eine kosmetische oder pharmazeutische Zusammensetzung, **dadurch gekennzeichnet, dass** sie in Verbindung mit jedem geeigneten Exzipienten einen Komplex auf der Basis von biologisch assimilierbarer Orthokieselsäure gemäß einem der Ansprüche 1 bis 3 umfasst.

6. Ein Nahrungszusatz, **dadurch gekennzeichnet, dass** er in Verbindung mit jedem alimentär geeigneten Exzipienten einen Komplex auf der Basis von biologisch assimilierbarer Orthokieselsäure gemäß einem der Ansprüche 1 bis 3 umfasst.
